# EUROPÄISCHE PATENTSCHRIFT

(11) **EP 1 543 784 B1**
(45) Veröffentlichungstag und Bekanntmachung des Hinweises auf die Patenterteilung: **15.10.2008**
(21) Anmeldenummer: 04026465.7
(22) Anmeldetag: 08.11.2004
(51) Int. Cl.: A61B 17/15

(54) **Resektionsschnittlehre und Verfahren zur Positionierung einer Resektionsschnittlehre**
Resection cutting jig and method for positioning the resection cutting jig
Gabarit de découpage et methode pour le positionnement du gabarit

(30) Priorität: 16.12.2003 DE 10358926
(43) Veröffentlichungstag der Anmeldung: 22.06.2005
(73) Patentinhaber: Mathys AG Bettlach, 2544 Bettlach (CH)
(72) Erfinder: Fankhauser, Christoph, 4500 Solothurn (CH); Supper, Walter, 2540 Grenchen (CH); Wehrli, Ulrich, Dr., 1789 Lugnorre (CH); Delfosse, Daniel, Dr., 3303 Jegenstorf (CH)
(74) Vertreter: Körfer, Thomas

(56) Entgegenhaltungen:
- EP-A- 1 302 167
- WO-A-01/91647
- WO-A-94/05212
- FR-A- 2 587 198
- FR-A- 2 838 627
- US-A- 5 681 316
- US-A1- 2002 133 162

## Beschreibung

Die Erfindung betrifft eine Resektionsschnittlehre für Gelenke des menschlichen oder tierischen Körpers.

Beispielsweise ist aus der DE 102 15 358 A1 eine Schnittlehre bekannt, welche auf eine Bänderspannvorrichtung aufsteckbar ist. Die Bänderspannvorrichtung wird zur Vorbereitung für die Implantierung eines Gelenksimplantats in ein Gelenk eingebracht und weist einen Grundkörper sowie Pratzen mit Knochenanlageflächen auf, wobei die Pratzen parallel zueinander verschiebbar sind. Die Schnittlehre wird auf Halterungen des Grundkörpers der Bänderspannvorrichtung aufgesetzt und gibt nach dem Spannen der Bänderspannvorrichtung die Schnittführung für die Knochenschnitte bei der prothetischen Versorgung eines Gelenks vor.

Nachteilig an der aus der DE 102 15 358 A1 bekannten Schnittlehre ist insbesondere, daß die Anbringung von Schnittebenen an einem erkrankten Gelenk zur Einbringung einer Prothese mehrere Montage- und Demontageschritte erfordert, welche unabhängig voneinander angesetzt werden müssen und dadurch weder eine genaue Positionierung und Ausrichtung noch eine reproduzierbare, genaue Schnittführung erlauben.

Weiterhin ist die bekannte Schnittlehre nicht zum Einsatz in Navigationssystemen geeignet.

Eine Resektionsschnittlehre gemäß dem Oberbegriff des Anspruchs 1 ist in der EP-A-1 302 167 gezeigt. Weiterhin ist eine Resektionsschnittlehre in der FR-A-2838627 gezeigt.

Der Erfindung liegt demnach die Aufgabe zugrunde, eine Resektionsschnittlehre zu schaffen, welche bzw. welches eine voreinstellbare, nachjustierbare und nachkontrollierbare Schnittführung bei der Vorbereitung und Durchführung der für die prothetische Versorgung eines Gelenkes benötigten Anschnitte ermöglicht.

Die Aufgabe wird bezüglich der Resektionsschnittlehre durch die Merkmale des Anspruchs 1 gelöst.

Die Resektionsschnittlehre umfaßt dabei ein Befestigungsteil und eine Aufnahmeeinheit, welche bezüglich des Befestigungsteils in drei voneinander unabhängigen Achsen schwenkbar ist, und

Weitere vorteilhafte Ausgestaltungen der Erfindung sind in den Unteransprüchen gekennzeichnet.

Vorteilhafterweise sind die translatorischen Freiheitsgrade und die rotatorischen Freiheitsgrade getrennt voneinander einstellbar.

Vorteilhafterweise weist die Aufnahmeeinheit ein gegenüber dem an dem Skeletteil angeordneten Befestigungsteil verdrehbares Verbindungsteil auf.

Weiterhin ist von Vorteil, daß die Aufnahmeeinheit eine Aufnahme für einen Knochenschnittblock aufweist, welche ihrerseits in zwei Richtungen gegenüber dem Verbindungsteil verschwenkbar ist.

Die drei Rotationsachsen der Aufnahmeeinheit stehen dabei senkrecht aufeinander, wobei die einzelnen Einstellungen separat voneinander erfolgen können.

Vorteilhafterweise wird die Aufnahmeeinheit jeweils nach der Einstellung eines der rotatorischen Freiheitsgrade in ihrer Position fixiert, so daß die weiteren Einstellungen diese Position in der Folge unbeeinflußt lassen.

Nach der Einstellung der rotatorischen Freiheitsgrade können translatorische Verschiebungen des Knochenschnittblocks relativ zur Aufnahmeeinheit bzw. zum Skeletteil erfolgen. Die Einstellung der rotatorischen und translatorischen Freiheitsgrade kann dabei nacheinander, aber auch gleichzeitig erfolgen.

Vorteilhafterweise ist der Knochenschnittblock dabei dreiteilig aufgebaut, wobei ein Schlitten relativ zu einem Oberteil in einer ersten Raumrichtung, das Oberteil gegenüber dem Unterteil in einer zweiten Raumrichtung und der Knochenschnittblock gegenüber der Aufnahme in einer dritten Raumrichtung verschieblich ist.

Ebenso wie die rotatorischen Freiheitsgrade können auch die translatorischen Freiheitsgrade in einfacher Weise durch Schrauben oder Klemmschrauben fixiert werden.

Weiterhin ist von Vorteil, daß der Knochenschnittblock mit einer Referenzbasis bestückt werden kann und auf diese Weise einfach und schnell mit einem Navigationssystem koppelbar ist.

Die Erfindung wird im folgenden anhand teilweise schematischer Darstellungen in der Zeichnung für die Vorbereitung der prothetischen Versorgung eines menschlichen Kniegelenks näher erläutert. In der Zeichnung zeigen:
- Fig. 1: eine schematische, perspektivische Ansicht einer erfindungsgemäßen Resektionsschnittlehre,
- Fig. 2: eine schematische, perspektivische Ansicht des Befestigungsteils und der Aufnahmeeinheit,
- Fig. 3: eine Aufsicht auf die am Knochen fixierte Resektionsschnittlehre, und
- Fig. 4: eine schematische, perspektivische Ansicht einer Referenzbasis für ein Navigationssystem, welches zur Verwendung in Kombination mit der erfindungsgemäßen Resektionsschnittlehre geeignet ist.

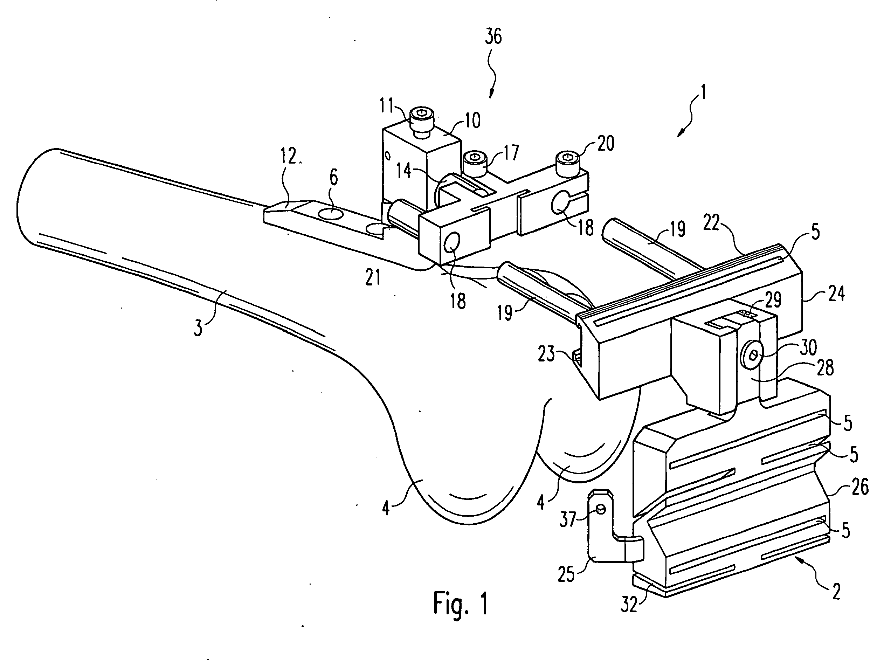

Fig. 1 zeigt in einer schematischen, perspektivischen Ansicht eine erfindungsgemäß ausgestaltete Resektionsschnittlehre 1 mit einem Befestigungsteil 12 und einer Aufnahmeeinheit 36. Das Befestigungsteil 12 ist dabei bereits in Anlage an einem Skeletteil 3 dargestellt, während ein Knochenschnittblock 2 noch nicht in die Aufnahmeeinheit 36 eingesetzt ist.

Bei dem in den Fig. 1 bis 3 jeweils dargestellten Skeletteil 3 handelt es sich um einen Oberschenkelknochen oder Femur, welcher zwei distale, einem nicht weiter dargestellten Kniegelenk zugewandte Kondylen 4 aufweist. Zur prothetischen Versorgung eines Kniegelenks werden dabei eine oder beide Kondylen 4 durch Resektion abgetragen und eine Prothese implantiert. Die Resektion erfolgt dabei entlang verschiedener Schnittebenen, welche durch die Form des Implantats vorgegeben werden. Zur korrekten Anpassung des Implantats ist es von entscheidender Bedeutung, daß die Schnitte exakt geführt und der Knochenschnittblock 2, durch welchen die Schnittebenen definiert sind, genauestens positionierbar ist. Die Positionierung erfolgt dabei vorzugsweise mit Hilfe eines Navigationssystems. Bisher bekannte Schnittlehren werden von Hand positioniert, wodurch eine höhere Ungenauigkeit resultiert und Nachbearbeitungen zur Anpassung des Implantats notwendig sind. Dies ist zum einen aufwendig, außerdem verlängert sich die Operationsdauer.

Die Aufgabe des Befestigungsteils 12 und der Aufnahmeeinheit 36 besteht demnach darin, die korrekte Positionierung des Knochenschnittblocks 2 durch die Fixierung aller möglichen rotatorischen und translatorischen Freiheitsgrade zu gewährleisten.

Der Knochenschnittblock 2 weist mehrere Schnittführungen 5 auf, durch welche mit einer nicht näher dargestellten Säge die Schnitte geführt werden, die für die Anpassung des Implantats benötigt werden. Dabei werden eine oder beide Kondylen 4 unter verschiedenen Schnittwinkeln reseziert.

Durch die Aufnahmeeinheit 36 werden zunächst die rotatorischen Freiheitsgrade fixiert, während die translatorischen Freiheitsgrade durch den Knochenschnittblock 2 fixiert werden.

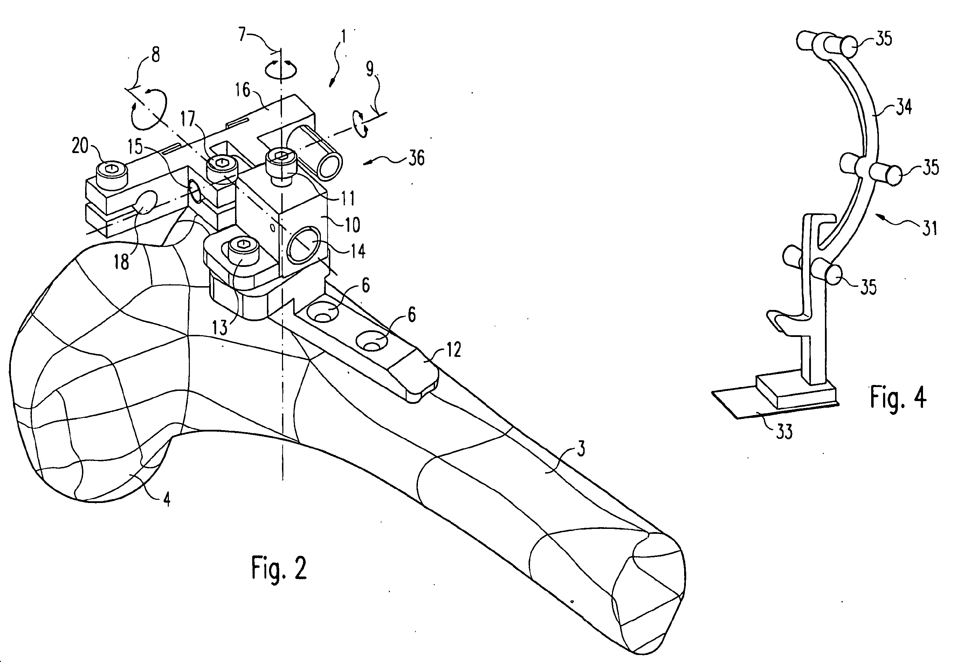

Betrachtet man zunächst Fig. 2, so ist erkennbar, daß das Befestigungsteil 12 auf dem Skeletteil 3 angesetzt und durch eine im Ausführungsbeispiel bikortikale Fixierung an dem Skeletteil 3 beispielsweise mittels zweier nicht dargestellter Schrauben durch zwei Bohrungen 6 fixiert wird. Eine bikortikale Fixierung gewährleistet dabei eine hohe Stabilität, welche dadurch erzielt wird, daß die Schraubfixierung des Befestigungsteils 12 durch beide Knochenlagen des röhrenförmigen Skeletteils 3 hindurch erfolgt.

In Fig. 2 sind zur Verdeutlichung der verschiedenen rotatorischen Freiheitsgrade der Aufnahmeeinheit 36 drei Achsen 7, 8, 9 eingezeichnet. Die erste Achse 7 wird dabei im Folgenden als Varus-Valgus-Achse 7 bezeichnet. Die Einstellung der korrekten Position eines mit dem Befestigungsteil 12 verbundenen Verbindungsteils 10 der Aufnahmeeinheit 36 erfolgt dabei mittels Rotation um eine Achse, welche parallel zur Schraube 11 verläuft, relativ zu dem Befestigungsteil 12. Die Schraube 11 dient dabei, wie weiter unten beschrieben, der Fixierung des zweiten rotatorischen Freiheitsgrades. Der Knochenschnittblock 2, welcher in Fig. 2 der Übersichtlichkeit halber nicht dargestellt ist, wird entsprechend der Rotation des Verbindungsteils 10 mit verschwenkt. Zum Fixieren des zugehörigen ersten rotatorischen Freiheitsgrades wird eine Schraube 13 angezogen, welche die eingestellte Position des Verbindungsteils 10 relativ zum Befestigungsteil 12 fixiert.

Die zweite Achse 8, im Folgenden als Extern-Interne Achse 8 bezeichnet, verläuft durch eine Lagerachse 14, welche in dem Verbindungsteil 10 parallel zum Skeletteil 3 angeordnet ist. Das Verbindungsteil 10 ist um diese Lagerachse 14 relativ zum Befestigungsteil 12 schwenkbar, wodurch auch der Knochenschnittblock 2 mitverschwenkt werden kann. Nach der Einstellung der Position des Verbindungsteils 10 im zweiten rotatorischen Freiheitsgrad wird das Verbindungsteil 10 des Befestigungsteils 12 durch die Schraube 11, welche zugleich als Varus-Valgus-Achse 7 dient, fixiert.

Die dritte Achse 9, im folgenden als Flexions-Extensions-Achse 9 bezeichnet, verläuft durch einen Bolzen 15, durch welchen eine Aufnahme 16 für den Knochenschnittblock 2 am Verbindungsteil 10 der Aufnahmeeinheit 36 schwenkbar gelagert ist. Bei Verschwenkung der Aufnahme 16 ist somit ebenfalls eine Mitverschwenkung des Knochenschnittblocks 2 möglich. Nach korrekter Positionierung der Aufnahme 16 relativ zum Verbindungsteil 10 des Befestigungsteils 12 wird diese durch eine weitere Schraube 17 fixiert. Nach diesem Schritt ist die Positionierung der Aufnahmeeinheit 36 bzw. dadurch auch des Knochenschnittblocks 2 in allen rotatorischen Freiheitsgraden abgeschlossen.

Betrachtet man nun nochmals Fig. 1, sind die weiteren Einstellmöglichkeiten des Knochenschnittblocks 2 in den verschiedenen translatorischen Freiheitsgraden ersichtlich.

Der Knochenschnittblock 2 wird zunächst mit zwei Stiften 19 in Führungen 18 in der Aufnahme 16 der Aufnahmeeinheit 36 eingeschoben. Eine der Führungen 18 ist dabei geschlitzt ausgebildet und weist eine Fixierungsschraube 20 auf, durch welche der Stift 19 in der Aufnahme 16 durch Klemmen fixiert wird. Die zweite Führung 18 weist eine Führungshülse 21 auf, damit der darin befindliche Stift 19 gerade geführt wird und es nicht zu einem Verkanten oder Verkippen des Knochenschnittblocks 2 kommen kann.

Durch die Führung der Stifte 19 in den Führungen 18 ist der erste translatorische Freiheitsgrad in proximal-distaler Richtung definiert. Durch Verschieben des kompletten Knochenschnittblocks 2 an das Skeletteil 3 heran oder von diesem weg kann dieser positioniert werden. Mittels der Fixierungsschraube 20 wird die Position des Knochenschnittblocks 2 fixiert.

Die Stifte 19 sind an einem Schlitten 22 ausgebildet, welcher in einer Führungsschiene 23 eines Oberteils 24 des Knochenschnittblocks 2 verschieblich geführt ist. Die Position des Schlittens 22 bedingt die Position des Knochenschnittblocks 2 relativ zur Aufnahmeeinheit 36 bzw. zum Skeletteil 3 und somit den zweiten translatorischen Freiheitsgrad in medial-lateraler Richtung. Durch Verschiebung des Schlittens 22 kann der Knochenschnittblock 2 beliebig in medial-lateraler Richtung verschoben werden. Eine separate Fixierung dieser Position ist nicht notwendig, da in einem dritten Schritt der dritte translatorische Freiheitsgrad eingestellt und fixiert wird und gleichzeitig die endgültige Fixierung des Knochenschnittblocks 2 am Skeletteil 3 erfolgt.

Dieser dritte Schritt der Einstellung erfolgt durch eine Verschiebung eines Unterteils 26 des Knochenschnittblocks 2 relativ zum Oberteil 24 des Knochenschnittblocks 2. Ein an dem Unterteil 26 ausgebildeter Fortsatz 28 ist dabei verschieblich in einer Führung 29 des Oberteils 24 gelagert, so daß eine anterio-posteriore Verschiebung des Unterteils 26 relativ zum Oberteil 24 entsprechend dem dritten translatorischen Freiheitsgrad erfolgen kann. Die Fixierung erfolgt mittels einer Klemmschraube 30, welche an dem Fortsatz 28 vorgesehen ist.

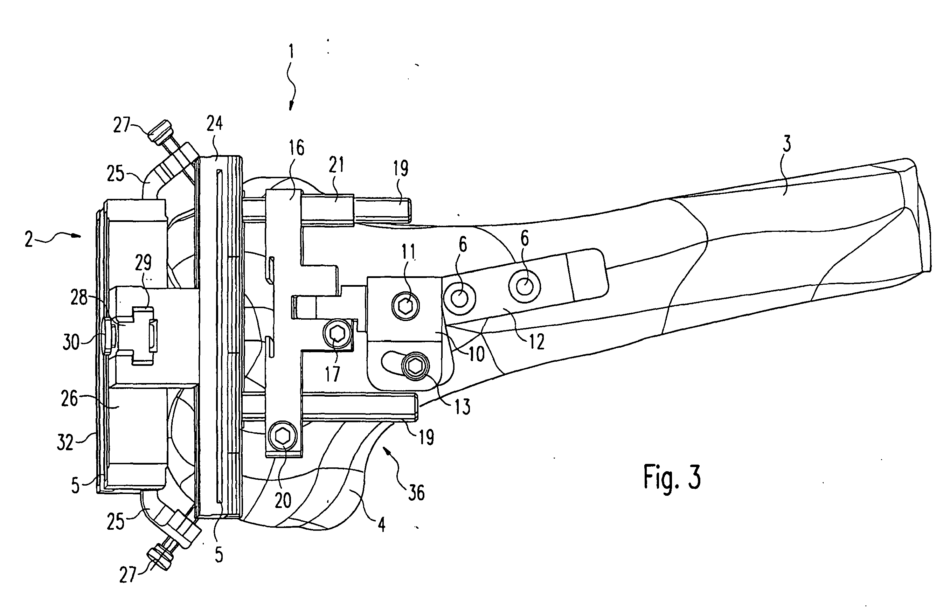

Zur endgültigen Fixierung des Knochenschnittblocks 2 in der korrekten Position werden durch Bohrungen 37 in zwei Laschen 25, welche medial und lateral an dem Unterteil 26 des Knochenschnittblocks 2 ausgebildet sind und in Anlage an das Skeletteil 3 gebracht werden, jeweils ein Fixationsnagel 27, wie in Fig. 3 ersichtlich, eingeschlagen, wodurch der Knochenschnittblock 2 am Skeletteil 3 fixiert ist.

Somit ist es mit der erfindungsgemäß ausgestalteten Resektionsschnittlehre 1 möglich, nacheinander und unabhängig voneinander alle rotatorischen und translatorischen Freiheitsgrade einzustellen und zu fixieren, um so eine hochpräzise Schnittführung zu erlauben. Alle Schnitte können mit einem Knochenschnittblock 2 ohne die Notwendigkeit weiterer Einstellschritte bzw. weiterer Schnittlehren erfolgen. Dadurch wird die Nachbearbeitung des Skeletteils 3 überflüssig, wodurch die Operationsdauer wesentlich verkürzt werden kann. Weiterhin ist die Einbindung in ein Navigationssystem möglich, wie anhand von Fig. 4 kurz beschrieben wird.

In Fig. 4 ist in einer schematischen perspektivischen Ansicht eine Referenzbasis 31 für den Knochenschnittblock 2 dargestellt, welche mit einem Fuß 33 in eine Aufnahme 32 am Unterteil 26 des Knochenschnittblocks 2 einschiebbar ist. Die Referenzbasis 31 weist einen bogenförmigen Arm 34 auf, an welchem mehrere, im Ausführungsbeispiel drei Reflektoren 35 angeordnet sind, welche durch eine entsprechende Vorrichtung des Navigationssystems abtastbar und somit in ihrer räumlichen Lage erfaßbar sind. Die Fixierung der Referenzbasis 31 am Knochenschnittblock 2 kann beispielsweise mittels einer nicht weiter dargestellten Feder erfolgen.

Die Erfindung ist nicht auf das dargestellte Ausführungsbeispiel beschränkt und läßt sich auch für andere Gelenke anwenden. Insbesondere kann die Einstellung der rotatorischen und translatorischen Freiheitsgrade nacheinander, aber auch gleichzeitig erfolgen.

## Patentansprüche

1. Resektionsschnittlehre (1) zur Resektion von Skeletteilen (3), insbesondere eines Gelenks, des menschlichen oder tierischen Körpers zur Vorbereitung der prothetischen Versorgung des Skeletteils (3),
wobei die Resektionsschnittlehre (1) ein an dem Skeletteil (3) fixiertes Befestigungsteil (12) und eine Aufnahmeeinheit (36) mit einem Verbindungsteil (10) für einen daran fixierbaren Knochenschnittblock (2) umfasst, **dadurch gekennzeichnet, dass**
der Knochenschnittblock (2) über die Aufnahmeeinheit (36) um drei voneinander unabhängige Achsen (7, 8, 9) gegenüber dem Befestigungsteil (12) verschwenkbar ist und
eine Aufnahme (16) der Aufnahmeeinheit (36) um eine durch eine Lagerachse (14) verlaufende zweite Achse (8) in intern-externer Richtung gegenüber dem Verbindungsteil (10) verschwenkbar ist.

2. Resektionsschnittlehre nach Anspruch 1,
**dadurch gekennzeichnet,**
**dass** der Knochenschnittblock (2) so an der Aufnahmeeinheit (36) angeordnet ist, dass er in drei voneinander unabhängigen Raumrichtungen verschieblich ist.

3. Resektionsschnittlehre nach Anspruch 1,
**dadurch gekennzeichnet,**
**dass** das Verbindungsteil (10) und das Befestigungsteil (12) zueinander verdrehbar angeordnet sind.

4. Resektionsschnittlehre nach Anspruch 3,
**dadurch gekennzeichnet,**
**dass** das Verbindungsteil (10) der Aufnahmeeinheit (36) um eine erste Achse (7) in Varus-Valgus-Richtung gegenüber dem Befestigungsteil (12) verschwenkbar ist.

5. Resektionsschnittlehre nach Anspruch 4,
**dadurch gekennzeichnet,**
**dass** das Verbindungsteil (10) gegenüber dem Befestigungsteil (12) durch eine Schraube (13) in seiner Position fixierbar ist.

6. Resektionsschnittlehre nach einem der Ansprüche 1 bis 5,
**dadurch gekennzeichnet,**
**dass** die Lagerachse (14) parallel zu dem Skeletteil (3) angeordnet ist.

7. Resektionsschnittlehre nach einem der Ansprüche 1 bis 6,
**dadurch gekennzeichnet,**
**dass** die Aufnahme (16) gegenüber dem Verbindungsteil (10) durch eine Schraube (11) in ihrer Position fixierbar ist.

8. Resektionsschnittlehre nach einem der Ansprüche 1 bis 7,
**dadurch gekennzeichnet,**
**dass** die Aufnahme (16) für den Knochenschnittblock (2) relativ zu dem Verbindungsteil (10) um eine durch einen Bolzen (15) verlaufende dritte Achse (9) in Flexions-Extensions-Richtung schwenkbar ist.

9. Resektionsschnittlehre nach Anspruch 8,
**dadurch gekennzeichnet,**
**dass** die Aufnahme (16) gegenüber dem Verbindungsteil (10) durch eine Schraube (17) in ihrer Position fixierbar ist.

10. Resektionsschnittlehre nach einem der Ansprüche 1 bis 9,
**dadurch gekennzeichnet,**
**dass** die Achsen (7, 8, 9) senkrecht zueinander stehen.

11. Resektionsschnittlehre nach einem der Ansprüche 1 bis 10,
**dadurch gekennzeichnet,**
**dass** das Befestigungsteil (12) durch zwei Bohrungen (6) mit dem Skeletteil (3) verbunden ist.

12. Resektionsschnittlehre nach Anspruch 11,
**dadurch gekennzeichnet,**
**dass** die Verbindung mittels bikortikaler Verschraubung erfolgt.

13. Resektionsschnittlehre nach einem der Ansprüche 1 bis 12,
**dadurch gekennzeichnet,**
**dass** der Knochenschnittblock (2) fünf Schnittführungen (5) aufweist.

14. Resektionsschnittlehre nach einem der Ansprüche 1 bis 9,
**dadurch gekennzeichnet,**
**dass** die Aufnahme (16) für den Knochenschnittblock (2) zwei Führungen (18) aufweist.

15. Resektionsschnittlehre nach Anspruch 14,
**dadurch gekennzeichnet,**
**dass** eine der Führungen (18) geschlitzt ausgeführt ist.

16. Resektionsschnittlehre nach Anspruch 14 oder 15,
**dadurch gekennzeichnet,**
**dass** der Knochenschnittblock (2) zumindest zwei Stifte (19) aufweist.

17. Resektionsschnittlehre nach Anspruch 16,
**dadurch gekennzeichnet,**
**dass** die Stifte (19) in den Führungen (18) gemäß einem ersten translatorischen Freiheitsgrad in proximal-distaler Richtung verschieblich geführt sind.

18. Resektionsschnittlehre nach Anspruch 16 oder 17,
**dadurch gekennzeichnet,**
**dass** der Knochenschnittblock (2) in der Aufnahme (16) durch eine Fixierungsschraube (20) in der geschlitzten Führung (18) über einen der Stifte (19) fixierbar ist.

19. Resektionsschnittlehre nach einem der Ansprüche 16 bis 18,
**dadurch gekennzeichnet,**
**dass** einer der Stifte (19) zusätzlich in einer an der Aufnahme (16) ausgebildeten Führungshülse (21) geführt ist.

20. Resektionsschnittlehre nach einem der Ansprüche 16 bis 19,
**dadurch gekennzeichnet,**
**dass** die Stifte (19) an einem Schlitten (22) angeordnet sind.

21. Resektionsschnittlehre nach Anspruch 20,
**dadurch gekennzeichnet,**
**dass** der Schlitten (22) gemäß einem zweiten translatorischen Freiheitsgrad in einer Führungsschiene (23) in medial-lateraler Richtung verschieblich ist.

22. Resektionsschnittlehre nach Anspruch 21,
**dadurch gekennzeichnet,**
**dass** die Führungsschiene (23) in einem Oberteil (24) des Knochenschnittblocks (2) ausgebildet ist.

23. Resektionsschnittlehre nach Anspruch 22,
**dadurch gekennzeichnet,**
**dass** der Knochenschnittblock (2) ein Unterteil (26) aufweist, welches gemäß einem dritten Freiheitsgrad in anterio-posteriorer Richtung verschieblich an dem Oberteil (24) angeordnet ist.

24. Resektionsschnittlehre nach Anspruch 23,
**dadurch gekennzeichnet,**
**dass** das Unterteil (26) einen Fortsatz (28) aufweist, der in einer Führung (29) des Oberteils (24) geführt ist.

25. Resektionsschnittlehre nach Anspruch 23 oder 24,
**dadurch gekennzeichnet,**
**dass** das Unterteil (26) gegenüber dem Oberteil (24) durch eine Schraube (30) fixierbar ist.

26. Resektionsschnittlehre nach einem der Ansprüche 23 bis 25,
**dadurch gekennzeichnet,**
**dass** am Unterteil (26) der Knochenschnittblocks (2) zwei Fortsätze (28) angeordnet sind.

27. Resektionsschnittlehre nach Anspruch 26,
**dadurch gekennzeichnet,**
**dass** der Knochenschnittblock (2) durch Einstellung einer endgültigen Position mittels Fixationsnägeln (27) an dem Skeletteil (3) fixierbar ist.

28. Resektionsschnittlehre nach einem der Ansprüche 23 bis 27,
**dadurch gekennzeichnet,**
**dass** das Unterteil (26) des Knochenschnittblocks (2) eine Aufnahme (32) aufweist, in welche ein Fuß (33) einer Referenzbasis (31) einsteckbar ist.

29. Resektionsschnittlehre nach Anspruch 28,
**dadurch gekennzeichnet,**
**dass** die Referenzbasis (31) einen gebogenen Arm (34) aufweist.

30. Resektionsschnittlehre nach Anspruch 29,
**dadurch gekennzeichnet,**
**dass** an dem Arm (34) mehrere Reflektoren (35) angeordnet sind.

31. Resektionsschnittlehre nach Anspruch 30,
**dadurch gekennzeichnet,**
**dass** die Referenzbasis (31) mittels der Reflektoren (35) für ein Navigationssystem referenzierbar ist.

## Claims

1. Resection guide (1) for resection of skeletal parts (3), in particular of a joint, of the human or animal body in preparation for the prosthetic provision of the skeletal part (3), the resection guide (1) comprising a fixing part (12) fixed to the skeletal part (3) and a receptacle unit (36) with a connecting part (10) for a bone cutting block (2) fixable thereto, **characterised in that** the bone cutting block (2) is swivellable via the receptacle part (36) with respect to the fixing part (12) about three mutually independent axes (7, 8, 9), and a receptacle (16) of the receptacle unit (36) is swivellable in an internal-external direction with respect to the connecting part (10) about a second axis (8) passing through a bearing axis (14).

2. Resection guide according to Claim 1, **characterised in that** the bone cutting block (2) is arranged on the receptacle unit (36) in such a manner that it is displaceable in three mutually independent spatial directions.

3. Resection guide according to Claim 1, **characterised in that** the connecting part (10) and the fixing part (12) are arranged rotatably with respect to one another.

4. Resection guide according to Claim 3, **characterised in that** the connecting part (10) of the receptacle unit (36) is swivellable with respect to the fixing part (12) about a first axis (7) in the varus-valgus direction.

5. Resection guide according to Claim 4, **characterised in that** the connecting part (10) is fixable in its position with respect to the fixing part (12) by means of a screw (13).

6. Resection guide according to any one of Claims 1 to 5, **characterised in that** the bearing axis (14) is arranged parallel to the skeletal part (3).

7. Resection guide according to any one of Claims 1 to 6, **characterised in that** the receptacle (16) is fixable in its position with respect to the connecting part (10) by means of a screw (11).

8. Resection guide according to any one of Claims 1 to 7, **characterised in that** the receptacle (16) for the bone cutting block (2) is swivellable in the flexion-extension direction relative to the connecting part (10) about a third axis (9) passing through a pin (15).

9. Resection guide according to Claim 8, **characterised in that** the receptacle (16) is fixable in its position with respect to the connecting part (10) by means of a screw (17).

10. Resection guide according to any one of Claims 1 to 9, **characterised in that** the axes (7, 8, 9) are disposed perpendicularly to one another.

11. Resection guide according to any one of Claims 1 to 10, **characterised in that** the fixing part (12) is connected to the skeletal part (3) by means of two bores (6).

12. Resection guide according to Claim 11, **characterised in that** the connection is effected by means of bicortical screw fixation.

13. Resection guide according to any one of Claims 1 to 12, **characterised in that** the bone cutting block (2) has five cutting guides (5).

14. Resection guide according to any one of Claims 1 to 9, **characterised in that** the receptacle (16) for the bone cutting block (2) has two guides (18).

15. Resection guide according to Claim 14, **characterised in that** one of the guides (18) is slotted.

16. Resection guide according to Claim 14 or 15, **characterised in that** the bone cutting block (2) has at least two pins (19).

17. Resection guide according to Claim 16, **characterised in that** the pins (19) are guided displaceably in the guides (18) with a first translational degree of freedom in the proximal-distal direction.

18. Resection guide according to Claim 16 or 17, **characterised in that** the bone cutting block (2) is fixable in the receptacle (16) via one of the pins (19) by means of a fixing screw (20) in the slotted guide (18).

19. Resection guide according to any one of Claims 16 to 18, **characterised in that** one of the pins (19) is additionally guided in a guide bush (21) formed on the receptacle (16).

20. Resection guide according to any one of Claims 16 to 19, **characterised in that** the pins (19) are arranged on a carriage (22).

21. Resection guide according to Claim 20, **characterised in that** the carriage (22) is displaceable in a medial-lateral direction in a guide rail (23) with a second translational degree of freedom.

22. Resection guide according to Claim 21, **characterised in that** the guide rail (23) is formed in an upper part (24) of the bone cutting block (2).

23. Resection guide according to Claim 22, **characterised in that** the bone cutting block (2) has a lower part (26) which is arranged displaceably on the upper part (24) with a third degree of freedom in the anterior-posterior direction.

24. Resection guide according to Claim 23, **characterised in that** the lower part (26) has a projection (28) which is guided in a guide (29) of the upper part (24).

25. Resection guide according to Claim 23 or 24, **characterised in that** the lower part (26) is fixable with respect to the upper part (24) by means of a screw (30).

26. Resection guide according to any one of Claims 23 to 25, **characterised in that** two projections (28) are arranged on the lower part (26) of the bone cutting block (2).

27. Resection guide according to Claim 26, **characterised in that** the bone cutting block (2) is fixable to the skeletal part (3) through setting of a final position by means of fixation nails (27).

28. Resection guide according to any one of Claims 23 to 27, **characterised in that** the lower part (26) of the bone cutting block (2) has a receptacle (32) in which a foot (33) of a reference base (31) is insertable.

29. Resection guide according to Claim 28, **characterised in that** the reference base (31) has a curved arm (34).

30. Resection guide according to Claim 29, **characterised in that** a plurality of reflectors (35) are arranged on the arm (34).

31. Resection guide according to Claim 30, **characterised in that** the reference base (31) can be referenced for a navigation system by means of the reflectors (35).

## Revendications

1. Gabarit de découpe par résection (1) pour la résection de parties de squelette (3), en particulier d'une articulation, du corps humain ou animal pour la préparation de la fourniture prothétique de la partie de squelette (3),
le gabarit de découpe par résection (1) comprenant un élément de fixation ( 12) fixé à la partie de squelette (3) et une unité de réceptacle (36) avec un élément de jonction (10) pour un bloc de découpe d'os (2) pouvant y être fixé,
**caractérisé en ce que**
le bloc de découpe d'os (2) au-dessus de l'unité de réceptacle (36) peut être basculé autour de trois axes indépendants les uns des autres (7, 8, 9) par rapport à l'élément de fixation (12) et un réceptacle (16) de l'unité de réceptacle (36) peut être basculé autour d'un deuxième axe (8) passant à travers un axe de palier (14) dans une direction interne-externe par rapport à l'élément de jonction (10).

2. Gabarit de découpe par résection selon la revendication 1,
**caractérisé en ce que**
le bloc de découpe d'os (2) est agencé au niveau de l'unité de réceptacle (36) de manière à pouvoir coulisser dans trois directions spatiales indépendantes les unes des autres.

3. Gabarit de découpe par résection selon la revendication 1,
**caractérisé en ce que**
l'élément de jonction (10) et l'élément de fixation (12) sont agencés de manière à pouvoir tourner l'un par rapport à l'autre.

4. Gabarit de découpe par résection selon la revendication 3,
**caractérisé en ce que,**
l'élément de jonction (10) de l'unité de réceptacle (36) peut être basculé autour d'un premier axe (7) dans une direction varus-valgus par rapport à l'élément de fixation (12).

5. Gabarit de découpe par résection selon la revendication 4,
**caractérisé en ce que,**
l'élément de jonction (10) peut être fixé dans sa position par rapport à l'élément de fixation (12) au moyen d'une vis (13).

6. Gabarit de découpe par résection selon l'une quelconque des revendications 1 à 5,
**caractérisé en ce que,**
l'axe de palier (14) est agencé de manière parallèle à la partie de squelette (3).

7. Gabarit de découpe par résection selon l'une quelconque des revendications 1 à 6,
**caractérisé en ce que,**
le réceptacle (16) peut être fixé dans sa position par rapport à l'élément de jonction (10) au moyen d'une vis (11).

8. Gabarit de découpe par résection selon l'une quelconque des revendications 1 à 7,
**caractérisé en ce que,**
le réceptacle (16) pour le bloc de découpe d'os (2) peut être basculé par rapport à l'élément de jonction (10) autour d'un troisième axe (9) passant à travers un boulon (15) dans une direction de flexion-extension.

9. Gabarit de découpe par résection selon la revendication 8,
**caractérisé en ce que,**
le réceptacle (16) peut être fixé dans sa position par rapport à l'élément de jonction (10) au moyen d'une vis (17).

10. Gabarit de découpe par résection selon l'une quelconque des revendications 1 à 9,
**caractérisé en ce que,**
les axes (7, 8, 9) sont perpendiculaires les uns par rapport aux autres.

11. Gabarit de découpe par résection selon l'une quelconque des revendications 1 à 10,
**caractérisé en ce que,**
l'élément de fixation (12) est raccordé à la partie de squelette (3) au moyen de deux alésage (6).

12. Gabarit de découpe par résection selon la revendication 11,
**caractérisé en ce que,**
la jonction s'effectue au moyen d'un raccord fileté bicortical.

13. Gabarit de découpe par résection selon l'une quelconque des revendications 1 à 12,
**caractérisé en ce que,**
le bloc de découpe d'os (2) présente cinq guidages de découpe (5).

14. Gabarit de découpe par résection selon l'une quelconque des revendications 1 à 9,
**caractérisé en ce que,**
le réceptacle (16) pour le bloc de découpe d'os (2) présente deux guidages (18).

15. Gabarit de découpe par résection selon la revendication 14,
**caractérisé en ce que,**
un des guidages (18) comprend des entailles.

16. Gabarit de découpe par résection selon la revendication 14 ou 15,
**caractérisé en ce que,**
le bloc de découpe d'os (2) présente au moins deux broches (19).

17. Gabarit de découpe par résection selon la revendication 16,
**caractérisé en ce que,**
les broches (19) dans le guidage (18) peuvent coulisser selon un premier degré de liberté translationnel dans la direction proximale-distale.

18. Gabarit de découpe par résection selon la revendication 16 ou 17,
**caractérisé en ce que,**
le bloc de découpe d'os (2) peut être fixé dans le réceptacle (16) au moyen d'une vis de fixation (20) dans le guidage à entailles (18) sur l'une des broches (19).

19. Gabarit de découpe par résection selon l'une quelconque des revendications 16 à 18,
**caractérisé en ce que,**
l'une des broches (19) est de plus guidée dans une gaine de guidage (21) formée au niveau du réceptacle (16).

20. Gabarit de découpe par résection selon l'une quelconque des revendications 16 à 19,
**caractérisé en ce que,**
les broches (19) sont agencées au niveau d'un chariot (22).

21. Gabarit de découpe par résection selon la revendication 20,
**caractérisé en ce que,**
le chariot (22) peut coulisser selon un deuxième degré de liberté translationnel dans un rail de guidage (23) dans une direction médiane-latérale.

22. Gabarit de découpe par résection selon la revendication 21,
**caractérisé en ce que,**
le rail de guidage (23) est formé dans une partie supérieure (24) du bloc de découpe d'os (2).

23. Gabarit de découpe par résection selon la revendication 22,
**caractérisé en ce que,**
le bloc de découpe d'os (2) présente une partie inférieure (26), laquelle est agencée pour coulisser au niveau de la partie supérieure (24) selon un troisième degré de liberté dans une direction antéro-postérieure.

24. Gabarit de découpe par résection selon la revendication 23,
**caractérisé en ce que,**
la partie inférieure (26) présente un prolongement (28) qui est guidé dans un guidage (29) de la partie supérieure (24).

25. Gabarit de découpe par résection selon la revendication 23 ou 24,
**caractérisé en ce que,**
la partie inférieure (26) peut être fixée en face de la partie supérieure (24) au moyen d'une vis (30).

26. Gabarit de découpe par résection selon l'une quelconque des revendications 23 à 25,
**caractérisé en ce que,**
deux prolongements (28) sont agencés au niveau de la partie inférieure (26) du bloc de découpe d'os (2).

27. Gabarit de découpe par résection selon la revendication 26,
**caractérisé en ce que,**
le bloc de découpe d'os (2) peut être fixé par ajustage d'une position définitive au moyen d'un clou de fixation (27) à la partie de squelette (3).

28. Gabarit de découpe par résection selon l'une quelconque des revendications 23 à 27,
**caractérisé en ce que,**
la partie inférieure (26) du bloc de découpe d'os (2) présente un réceptacle (32), dans lequel une patte (33) d'une base de référence (31) peut être introduite.

29. Gabarit de découpe par résection selon la revendication 28,
**caractérisé en ce que,**
la base de référence (31) présente un bras courbé (34).

30. Gabarit de découpe par résection selon la revendication 29,
**caractérisé en ce que,**
plusieurs réflecteurs (35) sont agencés au niveau du bras (34).

31. Gabarit de découpe par résection selon la revendication 30,
**caractérisé en ce que,**
la base de référence (31) peut être référencée au moyen de réflecteurs (35) pour un système de navigation.
